# EUROPÄISCHE PATENTSCHRIFT

(11) **EP 0 790 494 B1**
(45) Veröffentlichungstag und Bekanntmachung des Hinweises auf die Patenterteilung: **14.11.2001**
(21) Anmeldenummer: 97101601.9
(22) Anmeldetag: 03.02.1997
(51) Int. Cl.: G01N 1/34, G01N 33/49

(54) **Graphit-Vliese als funktionelle Schichten in diagnostischen Testkits**
Graphite fibre matrices as functional layers in diagnostic test kits
Matrices de fibres de graphite comme couches fonctionnelles dans des kits d'essais diagnostiques

(30) Priorität: 15.02.1996 DE 19605582
(43) Veröffentlichungstag der Anmeldung: 20.08.1997
(73) Patentinhaber: BAYER AG, 51368 Leverkusen (DE)
(72) Erfinder: Hildenbrand, Karlheinz, 47802 Krefeld (DE)

(56) Entgegenhaltungen:
- EP-A- 0 269 240
- WO-A-94/27140
- US-A- 4 477 575
- US-A- 5 290 420

## Beschreibung

Die vorliegende Erfindung betrifft die Verwendung von Graphit-Vliesen und Graphit-Geweben zum Abtrennen von zellulären Bestandteilen aus Blut und entsprechende Testmittel zur Analyse von Blutbestandteilen.

In der klinischen Chemie ist die Abtrennung von Serum oder Plasma aus Vollblut von überragender Bedeutung. Viele diagnostische Nachweisreaktionen von Blutkomponenten laufen nämlich erst nach Abtrennen der roten Blutkörperchen störungsfrei ab. Das gilt insbesondere für Farbreaktionen, die entweder reflektometrisch oder visuell aber auch elektrochemisch ausgewertet werden.

Die gebräuchlichste Form der Erythrocytenabtrennung ist die Zentrifugation. Diese ist jedoch insbesondere bei kleinen Blutproben problematisch, so daß hierfür eine ganze Reihe von Hilfsmitteln bekannt sind, wie beispielsweise in DE-AS 25 59 242 beschrieben.

Von besonderer Bedeutung ist die Verwendung spezieller Erythrocytenrückhaltesubstrate im Bereich der Vollblutanalyse mit Hilfe von Teststreifen, wie die Blutzuckerkontrolle unter home user-Bedingungen.

Als Stand der Technik haben sich hierbei mehrschichtige Testsysteme, bestehend aus einer Reagenzschicht und einer Erythrocyten-Abtrennzone bestehend aus mindestens einer oder mehreren, gegebenenfalls unterschiedlicher Glasfaserschichten bewährt (US 4 477 575).

Das Vollblut wird auf die Glasfaserschicht aufgetragen, wobei die Erythrocyten in dieser Schicht als Folge einer Agglutination adsorbiert werden, während Plasma bzw. Serum in die Reagenzschicht diffundieren, wo die durch Erythrocyten ungestörte Nachweisreaktion ablaufen kann.

Wie in EP 0 133 895 beschrieben, können sich in der Glasfaserschicht Hilfsreagenzien, wie bestimmte polare Farbstoffe befinden, die eine Koagulation bzw. Agglutination der Erythrocyten bewirken.

Die Glasfasern können, laut DE-OS 30 29 579 lose gestapelt sowie in Form von Papieren, Vliesen oder Filzen verarbeitet sein, wobei auch mit Glasfasern gefüllte Säulen mitbeansprucht werden. Die verwendeten Glasfasern können nach US-Patent 4 477 575 einen Durchmesser im Bereich von 0,2 bis 5,0 µm besitzen und im Dichtebereich von 0,1 bis 0,5 g/cm² liegen.

Die EP-Al-0 269 240 betrifft Kohlenstoff-Faserfilter, sowie Glasfaserfilter zur Bluttrennung, während die US-5,290,420 eine Kombination von Proben- und elektrochemischer Analysezelle offenbart, die von einer Kohlenstoff-Faserrolle als Elektrodenmaterial Gebrauch macht.

Nachteilig bei den Diagnosetestsystemen mit Erythrocyten-Rückhaltesubstraten aus Glasfaservliesen ist deren relativ hoher Bedarf an Vollblutvolumina, die bei den bekannten Reflotron® Glucose-Testsystemen bei ca. 10 µl liegen. Kleinere Blutmengen, beispielsweise 5 µl oder weniger sind jedoch insbesondere im Hinblick auf eine möglichst schmerzfreie Blutgewinnung von großem Vorteil.

Ein weiterer wichtiger Nachteil der Glasfaservliese ist deren geringe mechanische Festigkeit, die noch deutlich unter den Werten liegt, wie sie beispielsweise von dünnen Löschpapieren bekannt ist.

Eine maschinelle Bearbeitung, beispielsweise Schneiden oder Tränken mit konventionellen Maschinen, die gewisse Reißfestigkeiten voraussetzen, wird dadurch außerordentlich erschwert.

In WO 94/27140 sind Erythrocyten-Retentionsschichten aus porösen Membranmatrizen beschrieben, die beispielsweise Dextrane, Polylysine, Polybrene oder Protamine als Agglutinationsmittel enthalten. Im Vergleich zu den o.g. Glasfasersystemen sind diese Membranschichten jedoch komplizierter in der Herstellung und nicht so variabel bezüglich Schichtdicke, Aufnahmevolumen und Fließ- bzw. Transporteigenschaften, wobei insbesondere horizontale, chromatographieartige Transportfunktionen, welche zur Realisierung bestimmter Teststreifenformate (beispielsweise Fig. 4 in US 4 477 575) erforderlich sind, nicht realisiert werden können.

Ebenso wie bei den obengenannten weißen Glasfaserschichten kommt es nach Blutaufgabe zu einer intensiven roten Verfärbung der Rückhalteschichten. Bei der reflektometrischen Auswertung der darüberliegenden Reagenzschichten kann es aufgrund dieser roten Hintergrundfärbung zu unerwünschten Interferenzproblemen kommen.

Es wurde nun überraschenderweise gefunden, daß Vliese aus Graphit-Fasern die gestellten Anforderungen an die Erythrocytenabtrennfunktion hervorragend erfüllen können, ohne daß die oben erwähnten Nachteile auftreten.

Derartige Graphit-Vliese werden von der Firma SGL Carbon Group hergestellt, wobei sich die Type Sigrafil SPC 7011 für die erfindungsgemäßen Erythrocyten Abtrennschichten besonders bewährt hat.

Es handelt sich hierbei um schwarze, sehr reißfeste Vliese, bestehend aus Graphitfasern mit einem mittleren Faserdurchmesser von 7 µm, einem Flächengewicht von 30 g/m², einer Dicke von 0,5 mm und einem Bindemittelsystem aus vernetztem Polyvinylalkohol, dessen Anteil bei ca. 20 bis 24 Gew.-% liegt.

Ebenso geeignet zur Herstellung der erfindungsgemäßen Erythrocyten-Rückhalteschichten sind Gewebe, die ebenfalls aus Graphitfasern hergestellt werden können und unter dem Namen Sigratex® vertrieben werden.

Als Folge dieses sehr hydrophilen Polymerbinders zeichnen sich die Sigrafil® Graphitvliese darüber hinaus durch eine ausgezeichnete Benetzbarkeit aus. Ähnlich wie bei Glasfaservliesen ist sowohl in horizontaler als auch in vertikaler Richtung ein sehr rascher Flüssigkeitstransport zu beobachten. Ähnlich gute Ergebnisse konnten mit der Sigrafil Type SPC 7016 erzielt werden, die sich von der oben genannten Type 7011 durch eine höhere Schichtdicke (0,8 mm) unterscheidet.

Weitere geeignete Vliese, die für die Erythrocytenabtrennung in Frage kommen, sind die Sigratherm® -Papiere, die aus Kohlenstoff-Stapelfasern hergestellt werden und ebenfalls von SGL Carbon Group vertrieben werden.

Insbesondere zu erwähnen sind die hohen Reißfestigkeiten von ca. 160 N/5 cm dieser Graphitvliese, so daß keine Verarbeitungsprobleme mit konventionellen Verarbeitungsmaschinen, die gewisse Reißfestigkeiten voraussetzen, bestehen.

Die Abtrennung der Erythrocyten mit Hilfe der beschriebenen Graphit-Vliese, deren mittlerer Faserdurchmesser bei 7 µm liegt, ist insofern als überraschend zu bezeichnen, als nach USP 4 777 575 Glasfaservliese mit Faserdurchmessern in diesem Bereich für die Erythrocytenabtrennung nicht mehr geeignet sein sollten.

So ist in Tab. 2 aus USP 4 477 575 zu ersehen, daß Glasfaservliese mit Faserdurchmessern von mehr als 2 µm für die Plasma- bzw. Serumseparation nicht mehr in Frage kommen.

Zwei weitere wichtige Vorteile der erfindungsgemäßen Graphitvlies Erythrocyten-Rückhaltesubstanz resultieren aus deren schwarzer Farbe. Nach der Blutaufgabe, beispielsweise auf ein zweischichtiges Testsystem aus Rückhalteschicht und Reagenzmembran, können die im Graphitvlies zurückgehaltenen roten Erythrocyten visuell kaum noch erkannt werden, was als ästhetischer Vorteil zu bewerten ist. Dieser Vorteil spiegelt sich auch bei der reflektrometrischen Auswertung der Farbreaktion in der Reagenzmembran wieder, weil im Gegensatz zu den konventionellen Systemen (rot gefärbte, erythrocytenhaltige Glasfaserschichten als Hintergrund) keine negativen reflektometrische Interferenzen resultieren können.

Die erfindungsgemäßen mit bekannten Agglutinationsmitteln, wie Lectinen imprägnierten Graphitvliese können als ein- oder mehrschichtige Systeme zur Anwendung kommen. Bei mehrschichtigen Rückhaltesubstraten können alle oder auch nur einzelne Graphit-Vliesschichten mit einem oder verschiedenen Agglutinationsmitteln imprägniert werden. Bei mehrschichtigen Rückhaltesubstanzen können neben Graphitvliesen als elementare Komponente auch weitere poröse Schichten, wie Polyvinylalkoholvliese oder mono- bzw. multifile Gewebe enthalten sein..

Wesentlich ist nur, daß der Hauptanteil der agglutinierten Erythrocyten in einer Graphitvliesschicht zurückgehalten wird und daß vorzugsweise die oberste Schicht (Blutaufgabe) sowie die der Reagenzmembran nächstliegende Schicht aus Graphit-vlies besteht.

Eine weitere typische Eigenschaft der Graphitvliese ist deren elektrische Leitfähigkeit, die im für Carbonfasern typischen Bereich von einigen Ohm liegt.

Durch die Kombination aus elektrischer Leitfähigkeit mit der hervorragenden Eigenschaft des horizontalen Flüssigkeitstransports lassen sich im Hinblick auf elektrochemische Sensorensysteme Schichten mit bifunktionellen Funktionen aufbauen. Wie in Beispiel 2 detaillierter dargestellt, kann eine Graphitvliesschicht in einem amperometrischen Testformat gleichzeitig die Funktion der flüssigkeitsansaugenden Mikrokapillare (ein bei Biosensoren beliebter Applikationsmodus (Eur. Pat. Appl. 0 471 986)) ausüben sowie gleichzeitig als Referenzelektrode fungieren.

Die in dieser Patentbeschreibung angesprochenen Aufgaben werden erfindungsgemäß durch ein mehrschichtiges Testmittel nach dem kennzeichnenden Teil des Anspruchs 1 gelöst, wobei die Probenaufgabeschicht des erfindungsgemäßen mehrschichtigen Testmittels, gemäß Anspruch 2, zusätzlich noch Reagenzien und gegebenenfalls auch, wie in Anspruch 3 beschrieben, Agglutinations- und Retentionsmittel für rote Blutkörperchen enthalten kann.

Darüber hinaus lösen die Graphitvliese oder Graphitgewebe durch ihre Doppelfunktion (gleichzeitige Funktion als flüssigkeitsansaugenden Mikrokapillare, sowie als Referenzelektrode) erfindungsgemäß eine weitere Aufgabe, durch die Verwendung gemäß Anspruch 4.

### Beispiele

### Beispiel 1

### Visueller Blutzucker-Test

### a) Erythrocyten-Retentionsschicht

Ein Graphit-Vlies (SPC 7011 Fa. SGL) wurde mit folgender Lösung imprägniert und anschließend mit Warmluft getrocknet:

| | |
|---|---|
| 0,04 g | Lectin (aus Kartoffeln, SERVA, Potato Lectin) wurden in |
| 1,5 ml | der folgenden Chremophor EL-Tensidlösung gelöst: |
| | |
| 97,60 g | Wasser |
| 0,85 g | Cremophor EL (Sigma, C 5135) |
| 2,20 g | HEPES-Puffer 0,5 m (Sigma, H 7006); pH 7,5 |

### b) Reagenzschicht

### - Herstellung der Membranmatrix (analog DE-AS 4 308 150)

| | |
|---|---|
| Aus | |
| 20,0 g | Dralon L (Polyacrylnitril, Bayer AG) |
| 80,0 g | Ultrason E (Polyethersulfon, BASF) |
| 20,0 g | Aerosil 200 (hochdisperse Kieselsäure, Degussa) |
| 90,0 g | Pluriol P 900 (Polypropylenglykol, BASF) |
| 413,4 g | N-Methyl-2-pyrrolidon (NMP, Riedel deHäen) |

wurde mit einem hochtourigen Rührer eine Gießlösung hergestellt, die nach Entgasen mit Hilfe eines Rakels auf ein Polyestervlies (FO 2402, Fa. Freudenberg) beschichtet und in Wasser (40°C) koaguliert wurde.

Es wurde eine poröse, trägergestützte Membran (mittlere Porengröße ca. 5 bis 8 µm) erhalten, die nach dem Trocknen für die nachfolgende Imprägnierung eingesetzt wurde:

### - Imprägnierung der Polymerblend-Membran:

| Tränklösung 1 : | |
|---|---|
| 15,0 mg | Peroxidase (618 U/mg) |
| 50,0 mg | 3-Methyl-2-benzothiazolinon-Hydrazon-Hydrochlorid |
| 7,5 ml | Methanol |
| 7,5 ml | Kaliumphosphatpuffer 0,1 mol/l pH 7,0 |

| Tränklösung 2: | |
|---|---|
| 100,0 mg | 3-Dimethylaminobenzoesäure |
| 7,5 ml | Methanol |
| 7,5 ml | Kaliumphosphatpuffer 0,1 mol/l pH 7,0 |

| Tränklösung 3: | |
|---|---|
| 77,25 mg | Glucoseoxidase (151 U/mg) |
| 15,0 ml | Kaliumphosphatpuffer 0,1 mol/l pH 7,0 |

Nach Trocknung mit Warmluft wurde die Reagenzmembran erhalten. Entsprechend der folgenden Abbildung wurde ein mehrschichtiges Testsystem für den Blutzuckernachweis aufgebaut.

### Sandwich-Aufbau: Ablesen der Farbreaktion von der Unterseite

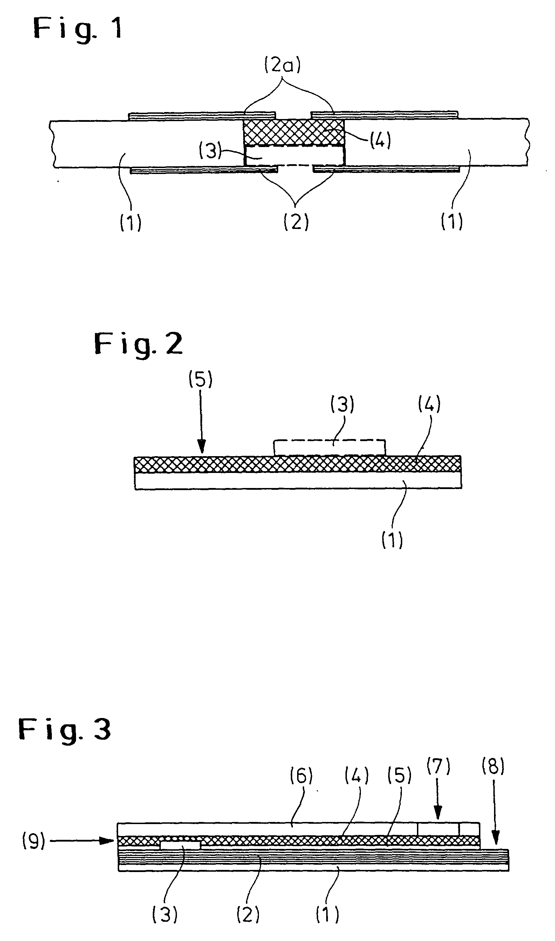

Der Aufbau wird in der Fig. 1 dargestellt.
1: Teststreifenhalter durchlocht
2: Transparente, durchlochte Abdeckfolien
3: Reagenzmembran
4: Imprägniertes Graphit-Vlies

Die Aufgabe des Blutes erfolgte über die Öffnung bei 2a), auf der gegenüberliegenden Seite konnte nach wenigen Sekunden eine durch Erythrocyten ungestörte blaue Farbreaktion, die mit der Glucosekonzentration des Vollblutes korrelierte, beobachtet werden.

Die Blutbestandteile waren vollständig in das Graphitvlies eingedrungen, so daß nach der Reaktion wegen der schwarzen Vliesfarbe keinerlei "roten Rückstände" zu erkennen waren, was im Vergleich zum Stand der Technik als ästhetischer Vorteil zu werten ist. Die aufzugebende Blutmenge konnte bei Schichtdurchmessern (Reagenzmembran, Graphitvlies) von 5 mm auf 5 µl begrenzt werden.

### Capillar Fließsystem: Beobachten der Farbreaktion von der Oberseite

Der Aufbau wird in der Fig. 2 dargestellt.
1: Teststreifenhalter
2: Imprägniertes Graphitvlies
3: Reagenzmembran

Die Aufgabe des Vollblutes erfolgte bei 5, wenige Sekunden später konnte an der Oberfläche der Reagenzmembran 3 eine durch rote Erythrocyten ungestörte Farbreaktion beobachtet werden.

Mit steigenden Glucosekonzentrationen der Testlösungen wurden zunehmende blaue Farbintensitäten beobachtet.

### Beispiel 2

### Visueller Cholesterin-Test

Die Herstellung der Erythrocyten-Rückhalteschicht der Reagenzmembran sowie der Teststreifen-Aufbau erfolgte analog zu Beispiel 1.

### Tränkrezeptur für die Reagenz-Membran:

| Tränklösung 1: | |
|---|---|
| 15,0 mg | Peroxidase (618 U/mg) |
| 50,0 mg | 3-Methyl-2-benzothiazolinon-Hydrazon-Hydrochlorid |
| 7,5 ml | Methanol |
| 7,5 ml | Kaliumphosphatpuffer 0,1 mol/l pH 7,0 |

| Tränklösung 2: | |
|---|---|
| 100,0 mg | 3-Dimethylaminobenzoesäure |
| 7,5 ml | Methanol |
| 7,5 ml | Kaliumphosphatpuffer 0,1 mol/l pH 7,0 |

| Tränklösung 3: | |
|---|---|
| 16,0 mg | Cholesterinoxidase (24,3 U/mg) |
| 9,0 mg | Cholesterinesterase (118 U/mg) |
| 500 µl | Kaliumphosphatpuffer 0,1 mol/l pH 7,0 |

Die Trocknung der Reagenzmembran erfolgte bei 45°C im Umlufttrockenschrank.

Beim Testen mit Vollblut (Proben mit unterschiedlichen Cholesteringehalten) wurden blaue Farbreaktionen beobachtet, deren Farbintensitäten mit den jeweiligen Cholesterinkonzentrationen korrelierten.

### Beispiel 3

### Amperometrisches Testkit

Der Aufbau wird in der Fig. 3 dargestellt.
1 Trägerfolie (Basisfolie)
2: Leitfähige Schicht (beispielsweise Graphit, Gold oder Palladium)
3: Poröse Dielektrizitätsschicht (Membran oder Vlies)
4: Graphitvlies
5: Doppelklebeband
6: Deckfolie
7: Kontaktierung der Referenzelektrode
8: Kontaktierung der Arbeitselektrode
9: Aufgabe der Probeflüssigkeit

Mit Hilfe eines Potentiostaten, dessen Spannung auf 400 mV eingestellt und entsprechend der obigen Abbildung kontaktiert war, wurden chronamperometrische Messungen (Grundlagen s. beispielsweise Chem. in unserer Zeit 15 (1981) 21 ff) durchgeführt. Als Testlösungen, die über die Graphitvlies-Stirnkante (9) mit Hilfe einer Pipette aufgegeben wurden, dienten Ferri-/Ferrocyanat-Testlösungen, wobei die folgende Probeflüssigkeitsserie analysiert wurde:
1. K₃Fe (CN)₆ (Kaliumhexacyanoferrat) 200 mmol in Wasser (Stammlösung)
2. 2 mmol K₄Fe (CN)₆ in 198 mmol Stammlösung
3. 4 mmol K₄Fe (CN)₆ in 196 mmol Stammlösung
4. 6 mmol K₄Fe (CN)₆ in 194 mmol Stammlösung
5. 8 mmol K₄Fe (CN)₆ in 192 mmol Stammlösung
6. 10 mmol K₄Fe (CN)₆ in 190 mmol Stammlösung

Bei der chronamperometrischen Auswertung wurden entsprechend der Cotrell-Gleichung mit 1/t^{1/2} abfallende Stromkurven im µA Bereich gefunden, wobei mit steigenden K₄Fe (CN)₆ Konzentrationen, steigende Stromausbeuten erzielt wurden.

## Patentansprüche

1. Mehrschichtiges Testmittel, bestehend aus einer Reagenzschicht und einer Probenaufgabeschicht, **dadurch gekennzeichnet, daß** die Probeaufgabeschicht aus einem Graphitvlies oder Graphitgewebe besteht, und zudem vernetzten Polyvinylalkohol als Bindemittel enthält.

2. Testmittel gemäß Anspruch 1, worin die Probenaufgabeschicht zusätzlich noch Reagenzien enthält.

3. Testmittel gemäß den Ansprüchen 1 und 2, worin die Probenaufgabeschicht Substanzen enthält, die eine Agglutination und Retention der roten Blutkörperchen erwirken.

4. Verwendung von Graphitvliesen oder Graphitgeweben zur Abtrennung von zellulären Bestandteilen aus Blut und gleichzeit als Referenzelektrode bei elektrochemischen Biosensoren wobei die Graphitvliese oder Graphitgewebe vernetzten Polyvinylalkohol als Bindemittel enthalten.

## Claims

1. Multi-layer test agent comprising a reagent layer and a sample application layer, **characterized in that** the sample application layer is made of a graphite nonwoven or a graphite woven fabric and also comprises crosslinked polyvinyl alcohol as binder.

2. Test agent according to Claim 1, wherein the sample application layer additionally also comprises reagents.

3. Test agent according to Claims 1 and 2, wherein the sample application layer comprises substances which cause agglutination and retention of the red blood corpuscles.

4. Use of graphite nonwovens or graphite woven fabrics for removing cellular constituents from blood and at the same time as a reference electrode in electrochemical biosensors, the graphite nonwovens or graphite woven fabrics comprising crosslinked polyvinyl alcohol as binder.

## Revendications

1. Dispositif d'essai multicouche, constitué d'une couche à réactifs et d'une couche d'application d'un échantillon, **caractérisé en ce que** la couche d'application d'un échantillon est formée d'un voile ou d'un tissu de graphite, et contient en outre comme liant un polymère réticulé d'alcool vinylique.

2. Dispositif d'essai suivant la revendication 1, dans lequel la couche d'application d'un échantillon contient en plus des réactifs.

3. Dispositif d'essai suivant les revendications 1 et 2, dans lequel la couche d'application d'un échantillon contient des substances qui déclenchent une agglutination et la rétention des globules rouges du sang.

4. Utilisation de voiles ou tissus de graphite pour séparer des constituants cellulaires du sang et en même temps comme électrode de référence dans des biocapteurs électrochimiques, ces voiles ou tissus de graphite contenant comme liant un polymère réticulé d'alcool vinylique.
